# FASCICULE DE BREVET EUROPEEN

(11) **EP 0 933 077 B1**
(45) Date de publication et mention de la délivrance du brevet: **12.02.2003**
(21) Numéro de dépôt: 98403340.7
(22) Date de dépôt: 30.12.1998
(51) Int. Cl.: A61K 7/00, A61K 7/50

(54) **Patch nettoyant, révélateur de l'état de la peau**
Reinigungspflaster zur verbesserung des hautzustandes
Cleaning patch for improving the skin condition

(30) Priorité: 30.01.1998 FR 9801070
(43) Date de publication de la demande: 04.08.1999
(73) Titulaire: L'OREAL, 75008 Paris (FR)
(72) Inventeur: Gueret, Jean-Louis, 75018 Paris (FR)
(74) Mandataire: Boulard, Denis

(56) Documents cités:
- WO-A-94/02674
- FR-A- 2 750 050

## Description

La présente invention concerne un patch à action nettoyante. Outre l'action nettoyante, le patch peut avoir d'autres actions, notamment par la libération contrôlée sur l'épiderme d'un ou plusieurs composés pharmaceutiquement, cosmétiquement ou dermo-pharmaceutiquement actifs. De tels patchs sont efficaces notamment pour enlever les impuretés présentes sur la surface de la peau. De telles impuretés peuvent comprendre, des résidus liés à la pollution environnante, des cellules de peau mortes, des bouchons de sébum, des points noirs, des résidus de transpiration, etc.

Il est connu d'utiliser des patchs permettant, par transdermie, de faire pénétrer des composés actifs. De tels patchs présentent en général une structure comportant plusieurs couches successives dans l'ordre suivant : une première couche, dite couche support, généralement occlusive, c'est-à-dire constituée d'une matière imperméable, notamment à l'eau, à l'air, ou au composé actif de façon à empêcher la déterioration et/ou l'évaporation de ce dernier et faciliter la transdermie ; une seconde couche, dite couche de polymère, fixée sur la couche support et contenant le composé actif, cette couche pouvant venir directement au contact de la peau ; éventuellement, pour faciliter la fixation du patch sur la peau, une couche d'une matière adhésive appliquée à la surface de la couche de polymère et perméable au composé actif ; enfin, une couche détachable de protection, recouvrant de façon hermétique la couche de polymère, de façon à la protéger de toute contamination extérieure pendant le temps de stockage préalable à l'utilisation du patch.

Ainsi, par exemple, on connaît, en particulier selon le document US-A-5 232 707, une structure de patch constituée d'une couche support occlusive et comprenant, fixée sur cette dernière, une couche de polymère en une matrice d'un polymère de silicone incluant, à l'état dispersé, des particules d'huile.

Par ailleurs, il a été décrit dans le document WO96/14 822 un patch occlusif de forme adaptée pour la traitement des rides du genre précité, comportant dans une matrice anhydre un composé actif hydrosoluble sous forme de poudre, cette matrice pouvant contenir, en outre, une huile, utilisée en tant qu'agent de pénétration.

A titre d'exemple encore, le document FR-A-2 738 744 décrit un patch pour la libération contrôlée d'au moins un composé cosmétiquement ou dermo-pharmaceutiquement actif, comportant une couche, fixée sur une couche support, la couche étant constituée d'une matrice polymérique hydrophobe dans laquelle sont dispersées des particules du composé actif éventuellement instable en milieu oxydant et des particules d'au moins un agent hydro-absorbant, la couche réservoir étant anhydre.

Certains actifs tels que les kératolytiques, telle que l'acide salicylique, ou bien encore les α-hydroxyacides sont utilisés pour leur action "décapante", efficace pour débarrasser la peau de toutes ses impuretés, en particulier, les cellules de peau morte. D'autres actifs sont utilisés pour leur action d'absorption du sébum, de certains résidus gras ou de transpiration. Tous ces actifs, qui sont mis au contact de la peau, notamment par libération contrôlée sur ou dans l'épiderme, sont souvent relativement agressifs pour la peau. Il est donc souhaitable de ne pas les utiliser plus que nécessaire.

Il est connu d'incorporer de tels actifs dans des patchs du type de celui décrit ci-avant. Un inconvénient lié à tous ces dispositifs, tient au fait qu'il est difficile d'évaluer la fréquence d'application nécessaire et suffisante pour obtenir le résultat escompté, sans agresser inutilement la peau. En outre, le traitement utilisé n'est pas toujours le traitement qui convient le mieux au besoin de la peau traitée.

Aussi, est-ce un des objets de l'invention que de réaliser un patch à action nettoyante, et apte à renseigner la personne qui l'utilise, sur la fréquence d'application nécessaire et suffisante, et/ou sur le type de traitement nécessité par la peau.

C'est un autre objet de l'invention que de fournir un patch qui incorpore des actifs, qui outre leur action pour débarrasser la peau de ses impuretés, sous quelle que forme que ce soit, ont une autre action, notamment hydratante, adoucissante, ou cicatrisante.

La présente invention propose un patch apte à débarrasser la peau des impuretés qu'elle comporte, comprenant sur un support, une matrice polymérique contenant au moins un actif, la surface du patch destinée à être mise en contact avec la peau étant adhésive, caractérisée en ce qu'il comporte une couche colorée de manière à pouvoir, de visu, quantifier et/ou qualifier les impuretés extraites de la peau, et présentes sur ladite surface adhésive.

La surface adhésive est, de préférence, auto-adhésive à sec. Alternativement, la surface devient adhésive par adjonction d'eau, en formant un gel. Dans ce dernier cas de figure, le patch peut être posé sec sur une peau préalablement mouillée, ou mouillé sur une peau sèche.

L'actif peut être notamment un composé ayant une action permettant de détacher les impuretés de la peau (par exemple, les agents kératolytiques, qui décollent les cellules de peau morte), le prélèvement sur le patch pouvant se faire ensuite par action mécanique de l'adhésif. Il peut s'agir également d'un ou plusieurs composés contenus dans la matrice, et aptes à absorber directement les substances grasses (sébum) ou aqueuses (transpiration). Il peut s'agir aussi d'un mélange de tels actifs.

Ainsi, une fois décollé de la peau, le patch porte sur sa surface adhésive colorée, toutes les impuretés qui ont été extraites lors du traitement. De telles impuretés se présentent sous différentes formes, notamment des morceaux de peau morte, des points noirs, des résidus gras ou de transpiration. Ces impuretés peuvent être sous forme solide (généralement de couleur claire, notamment blanchâtre) ou dans le cas de graisses, ou de transpiration, former des auréoles (relativement claires) visibles sur la surface colorée du patch, et résultant du "pompage" par les composés absorbants présents dans la matrice. Dans le cas de particules solides, celles-ci sont prélevées par la couche adhésive, formée soit par la matrice, soit par une couche auxiliaire. En d'autres termes, l'adhésif en lui même peut avoir un rôle de "peeling" de la peau. Dans le cas de liquides, notamment gras, le transfert sur le patch peut se faire par absorption, grâce à la présence d'actifs absorbants dans la matrice.

La présence de la couche colorée selon l'invention, rend détectables de visu, et permet de quantifier (en nombre et en taille) et/ou qualifier les impuretés sur la surface colorée du patch. La quantité d'impuretés prélevées est représentative de la fréquence souhaitable d'application dudit patch. Ainsi, la présence d'une quantité importante de tels résidus indique à l'utilisatrice qu'elle doit appliquer le patch sur la base d'une fréquence relativement élevée (par exemple, tous les jours). Une quantité plus faible de telles impuretés indique que la fréquence d'utilisation doit être plus faible (par exemple, une fois par semaine). En d'autres termes, le patch selon l'invention permet de quantifier l'efficacité du traitement qu'il apporte. En outre, le type d'impuretés prélevées permet à l'utilisatrice de choisir au mieux le type de traitement nécessaire à sa peau.

Les actifs à effet nettoyant pouvant être utilisés selon l'invention, incluent notamment les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique. On peut utiliser également des actifs aptes à absorber le sébum, tels que la poudre de Kaolin, des particules de polyamide (notamment celles vendues sous la dénomination "ORGASOL" par la société ATOCHEM), ces dernières ayant en outre une action adoucissante au contact de la peau. On peut utiliser aussi des tanins ou de la terre de Sienne pour leurs propriétés absorbantes. On peut également utiliser des antibactériens, tels que le phosphate de clindamicyne, l'érythromycine ou les antibiotiques de la classe des tétracyclines. De tels actifs peuvent être utilisés en combinaison notamment avec des agents émollients, des agents adoucissants, notamment du miel ou des cires, et/ou des agents cicatrisants, notamment certains sels minéraux.

Avantageusement, la couche colorée est de couleur foncée, de manière à réaliser un contraste suffisant pour mettre en évidence les impuretés extraites de la peau, qui généralement sont de couleur claire. Typiquement, on peut utiliser du bleu foncé, du vert foncé, du rouge foncé, du noir, du marron, de l'orange, etc.

La matrice polymérique peut être constituée d'une matrice à base d'adhésif polyacrylique, polyvinylique, ou d'une matrice hydrophobe à base d'un polymère de silicone ou de polyuréthanne du type polyester polyuréthanne ou polyéther polyuréthanne. Le cas échéant, un catalyseur de réticulation ou l'agent de polyaddition est de préférence utilisé en une quantité telle que la réticulation ou la polyaddition ne soit pas complète, ceci de façon à ce que la couche de polymère présente en elle-même un caractère auto-adhésif satisfaisant pour ainsi avantageusement éviter que la couche support ne soit ultérieurement revêtue d'une couche adhésive. On peut utiliser également un polymère, par exemple un composé polyacrylique, en solution dans un solvant, notamment de l'acétate d'éthyl ou de l'alcool. L'adhérence du patch selon l'invention est typiquement comprise entre 50 g/cm² et 800 g/cm² (force exercée perpendiculairement au plan de la surface adhésive, nécessaire à son décollement de la peau), et de préférence entre 100 et 700 g/cm², et de préférence encore entre 300 et 600 g/cm². La surface de la matrice destinée à venir en contact avec la peau peut être lisse ou présenter des aspérités ou reliefs.

Avantageusement, la matrice formée par la couche de polymère peut contenir des particules d'au moins un agent hydro-absorbant dispersées de façon homogène dans ladite matrice. En effet, au contact de l'humidité de la peau, les particules de l'agent hydro-absorbant captent de l'eau, favorisant ainsi la solubilisation du composé actif solide, hydrosoluble. En quelque sorte, par cette solubilisation "in situ" de l'actif hydrosoluble, sa biodisponibilité est quasi instantanée, et toute interaction éventuelle avec les autres composés présents dans la couche polymérique est minimisée. L'humidité de la peau peut jouer le rôle de solubilisant de l'actif hydrosoluble d'autant plus, que la couche support du patch peut créer des conditions occlusives.

Parmi les agents hydro-absorbants pouvant être présents dans la matrice polymérique hydrophobe à l'état dispersé, on peut citer, de préférence, les polyacrylates réticulés superabsorbants à fort taux de gonflement dans l'eau, tels que ceux commercialisés par la Société NORSOLOR sous la dénomination *Aquakeep*® ; l'alcool polyvinylique ; les polymères carboxyvinyliques tels que ceux commercialisés par la Société GOODRICH sous les dénominations de "Carbopol"® ; les dérivés semi-synthétiques de la cellulose tels que la carboxyméthylcellulose ; les substances naturelles telles que les amidons, les gommes naturelles (gomme de guar, gomme arabique, gomme adragante), la caséine, les phytocolloïdes (carragénates, alginates, agar-agar), les fibres de coton et la gélatine.

On préfère tout particulièrement utiliser les polyacrylates réticulés superabsorbants dont la présence à l'état dispersé dans une matrice polymérique hydrophobe permet de pomper la transpiration par exemple, et favorise, après hydratation, un meilleur contact avec les particules des autres actifs présents le cas échéant dans la matrice.

L'agent hydro-absorbant tel que défini ci-dessus est présent, de préférence, dans une proportion allant d'environ 0,2 % à environ 20 % en poids, et plus particulièrement allant de 0,5 % à 10 % par rapport au poids total de la couche de polymère.

La couche support selon la présente invention peut être constituée de tout matériau approprié imperméable aux composés actifs contenus dans la couche de polymère adjacente. La couche support a non seulement pour fonction de supporter la couche de polymère mais également de servir de revêtement protecteur de celle-ci. Elle peut être de même dimension que la couche de polymère ou de dimension plus grande de telle sorte qu'elle s'étende au-delà la périphérie de la couche de polymère.

Le support peut être un support occlusif. A titre d'exemple, le support est constitué d'un matériau thermoplastique, choisi parmi les polyéthylènes haute et basse densité, les polypropylènes, les polychlorures de vinyle, les copolymères d'éthylène et d'acétate de vinyle, les polyesters et les polyuréthannes, ou d'un complexe de tels matériaux. Ces matériaux peuvent également être présents sous forme stratifiée avec au moins une feuille de métal telle qu'une feuille d'aluminium.

La couche support peut être de toute épaisseur appropriée qui procurera les fonctions de support et de protection souhaitées. De préférence, l'épaisseur de la couche support est comprise entre environ 20 µm et environ 1,5 mm. Avantageusement, la couche support est suffisamment flexible de manière à pouvoir épouser parfaitement le profil de la peau, et à ne pas provoquer chez l'utilisateur, une sensation d'inconfort.

De préférence toutefois, le support est non occlusif. Dans cette dernière hypothèse, on utilise avantageusement un support constitué d'un papier, d'un matériau thermoplastique poreux ou perforé, d'un tissé, d'un non tissé, d'un non tissé perforé.

De préférence, la couche colorée est formée de ladite matrice. La matrice polymérique peut être colorée par incorporation de pigments colorés n'opérant aucun transfert sur la peau. De tels pigments sont incorporés dans une concentration pouvant varier de 0,1 % à 10 % par rapport au poids total de la matrice, et de préférence, de 0,1% à 2 %. Le support peut être de couleur identique ou différente de celle de la matrice colorée. Avantageusement, le support est de couleur claire, ou voisine de celle de la peau, de manière à rendre le patch aussi discret que possible sur la peau. Selon une alternative, la couche colorée est formée du support en lui même, la matrice polymérique étant transparente.

Les pigments colorés peuvent être constitués notamment de pigments du type de ceux utilisés dans le domaine de l'alimentaire ou de la cosmétique, en particulier pour les rouges à lèvres ou les vernis à ongles. A titre d'exemple, on peut citer, seuls ou en combinaison, des pigments synthétiques, ou des pigments minéraux, notamment des pigments d'oxyde de titane, de zirconium ou de cérium, ainsi que les oxydes de zinc, de fer ou de chrome, et le bleu ferrique. On peut utiliser des pigments organiques, notamment le noir de carbone, les laques de baryum, strontium, calcium (DC Red N°7), aluminium. A titre d'exemple plus spécifique, on utilise un pigment portant la référence DC violet 2 K7014, commercialisé par KOHNSTAMM®.

Des particules d'un (ou plusieurs) composé(s) actif(s)solide(s), hydrosoluble(s), peuvent être incorporés dans la matrice polymérique. De tels actifs, utilisés seuls ou en combinaison, sont choisis parmi les actifs hydrophiles classiquement utilisés comme les agents antioxydants, anti-radicaux libres, hydratants, dépigmentants, liporégulateurs, anti-acnéiques, antiseborrheiques, anti-vieillissement, adoucissants, anti-rides, kératolytiques, anti-inflammatoires, rafraîchissants, cicatrisants, protecteurs vasculaires, antibactériens, antifongiques, antiperspirants, déodorants, conditionneurs de la peau, immunomodulateurs et nourrissants.

On peut utiliser notamment un ou plusieurs actifs choisis notamment parmi l'acide ascorbique et ses sels biologiquement compatibles, les enzymes, les antibiotiques tels que le phosphate de clindamycine, les composants à effet tenseur tels que les poudres de protéines de soja ou de blé, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines telles que la collagène et l'élastine, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone, etc..

La matrice peut également comporter au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

De tels actifs peuvent être incorporés à l'état solubilisé dans des huiles, utilisées seules ou en combinaison, parmi lesquelles on peut citer : les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, de pistache, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de germes de blé, de calophyllum, de sésame, de coriandre, de carthame, l'huile de passiflore, l'huile de rosa mosqueta, de macadamia, de pépins de fruits (raisin, cassis, orange, kiwi), de colza, de coprah, d'arachide, d'onagre, de palme, de ricin, de lin, de jojoba, de chia, d'olive ou de germes de céréales comme l'huile de germes de blé, l'huile de son de riz, l'huile de karité ; des acétylglycérides; des octanoates, décanoates ou ricinoléates d'alcools ou de polyalcools; des triglycérides d'acides gras; des glycérides ; l'huile de paraffine, de vaseline, le perhydrosqualène; des alcools gras (alcool stéarylique, alcool cétylique) et des acides gras (acide stéarique) et leurs esters ; les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA).

On peut encore citer les huiles hydrocarbonées partiellement fluorées ou les huiles perfluorées, et notamment les perfluoropolyéthers et les perfluoroalcanes.

La phase huileuse, c'est-à-dire les gouttelettes d'huile dispersées dans la couche de polymère, est présente dans une proportion allant de 0,1% à 30% en poids par rapport au poids total de la composition. De façon préférentielle, ce pourcentage est compris entre 5 et 25 %.

Alternativement, le composé actif liposoluble est incorporé dans une couche de polymère hydrophobe, sous forme de poudre ou de granulés.

Les patchs selon la présente invention peuvent, en outre, être protégés par la présence d'une couche détachable ou pelable de protection adjacente à la couche de polymère et/ou par un conditionnement dans un emballage approprié, notamment imperméable à l'eau et à la vapeur d'eau.

Lorsque la couche de polymère est protégée par une couche détachable de protection, celle-ci est enlevée au moment de l'utilisation. Elle peut être constituée en tout matériau imperméable au composé actif ainsi qu'à tout autre composant présent dans la matrice polymérique. Parmi les matériaux pouvant être utilisés, on peut citer de préférence une feuille de papier siliconée ou une feuille de matériau thermoplastique traitée pour la rendre anti-adhérente, par exemple à l'aide d'un vernis. De préférence, cette couche détachable de protection est constituée de polyéthylène. Avantageusement, la feuille de protection est constituée de deux parties se superposant sur une portion médiane du patch, de manière à en faciliter la pose sans que les doigts viennent en contact avec la matrice contenant le ou les actifs. Selon une alternative, la feuille de protection s'étend sur une surface supérieure à la surface du patch, et déborde au delà des limites de ce dernier, de manière à en favoriser le décollement.

De façon connue, les patchs selon la présente invention peuvent être découpés selon un contour approprié correspondant à la zone de surface de peau à traiter, par exemple sous forme de masque pour l'application sur le visage, notamment pour l'application sur le nez, les lèvres, les joues, la zone entre le nez et la lèvre supérieure, ou sur le front. Bien entendu, les patchs selon la présente invention peuvent être découpés sous toute autre forme nécessaire pour une application sur une zone déterminée du corps. En général, la taille d'un patch conforme à l'invention est comprise entre 0,25 cm² et 500 cm², et de préférence, entre 1 cm² et 30 cm².

Les patchs ainsi constitués et découpés peuvent être utilisés, après élimination de la couche détachable de protection, sur une surface de peau à traiter, en les appliquant directement sur une peau dont l'eau de transpiration permettra d'obtenir la libération désirée, le cas échéant, des composés actifs hydrophiles.

De préférence, un tel patch est utilisé pendant une durée d'application relativement courte, comparativement aux patchs de transdermie conventionnels. Sa durée d'application est de préférence comprise entre 30 s et 15 mn, et de préférence encore, comprise entre 30 s et 5 mn. Cette période d'application relativement courte autorise l'incorporation de charges ou d'actifs dans des concentrations plus élevées, le facteur adhérence étant moins critique que pour les patchs à longue durée d'application.

Avantageusement encore, le patch selon l'invention comprend au moins une zone de faible dimension, sous forme d'un onglet, apte à favoriser le décollement du patch.

Selon un mode de réalisation préféré de l'invention, on réalise un mélange à partir d'un polymère, notamment un polymère acrylique, solubilisé dans un solvant tel qu'un alcool, par exemple éthylique ou de l'acétate d'éthyl. On ajoute au mélange le ou les actifs ainsi que le ou les pigments colorants. Le mélange est déposé sur une surface non adhérente, notamment un polyéthylène siliconé. On provoque l'évaporation des solvants à une température comprise entre 30 °C et 100 °C. On obtient ainsi une couche adhésive, sur laquelle on dépose un support, notamment un non tissé.

Selon un autre mode de réalisation de la présente invention, dans le cas d'une matrice de silicone ou de polyuréthanne, la matrice polymérique constituant la couche de polymère est préparée par mélange intime sous agitation d'un prépolymère de silicone ou de polyuréthanne, du ou des pigments colorés et du ou des composés actifs.

Au mélange ainsi obtenu, on ajoute alors à basse température, en général à température ambiante, soit un catalyseur de réticulation si le prépolymère est un polymère de silicone, soit un isocyanate ou polyisocyanate si le prépolymère est un polyester-polyol ou un polyéther-polyol.

Le mélange est alors introduit dans une trémie et versé sur une feuille de polyéthylène par exemple, constituant la pellicule de protection détachable ou pelable du patch. En aval de la trémie est disposé une racle permettant d'égaliser l'épaisseur de la couche de polymère de la matrice polymérique, cette épaisseur étant généralement comprise entre 10 µm et 300 µm.

On applique ensuite, avant calandrage, une feuille de la couche support, occlusive ou non, qui peut être également une feuille de polyéthylène.

La polymérisation ou polyaddition est de préférence réalisée à température ambiante, ceci en vue de ne pas détériorer le ou les composés actifs. De façon générale, la polymérisation ou polyaddition est complète après environ 24 heures à température ambiante.

Après calandrage et avant que la polymérisation ou polyaddition ne soit complète, la structure composite obtenue peut être immédiatement découpée aux formes voulues, ce qui permet d'obtenir des bords pincés évitant tout phénomène de coulage.

### EXEMPLE 1

On réalise une composition comprenant en poids :
79,5% de polymère acrylique en solution dans de l'acétate d'éthyl ;
0,5 % de pigments d'oxyde de fer rouge ;
10 % d'ORGASOL® (particules de polyamide) ; et
10 % d'acide ascorbique.

Après homogénéisation, le produit est introduit dans une trémie et est étalé à l'aide d'une racle en une couche de 0,8 mm d'épaisseur sur une feuille de polyéthylène d'une épaisseur de 200 µm. Cette feuille peut être préalablement traitée en surface pour réduire son adhérence. L'ensemble est chauffé à 60° C de manière à permettre l'évaporation du solvant. La matrice adhésive est de couleur rouge foncé. On applique ensuite un film de polyéthylène (sans traitement anti-adhérent) de 30 µm d'épaisseur qui constitue la couche support du patch, et on procède au calandrage de l'ensemble. On obtient ainsi un ensemble comportant une couche support et une couche de polymère auto-adhésive formée d'une matrice polyacrylique colorée, cet ensemble comprenant en outre, une couche détachable de protection.

A partir de cet ensemble, on peut réaliser par découpe, différentes formes de patch en fonction des utilisations souhaitées. Les patchs après découpe sont ensuite conditionnés dans des sachets de polyéthylène. Lors de l'utilisation, le patch, après élimination de la couche détachable de protection, est appliqué directement sur le front, par exemple pendant une période de 5 minutes environ.

Un tel patch a une action blanchissante sur la peau, liée à la présence d'acide ascorbique. La présence d'ORGASOL® permet de pomper le sébum libéré par la peau. L'ORGASOL® a en outre une action adoucissante. Après décollement, l'observation de la face rouge du patch montre la présence notamment d'auréoles visibles, formées par le sébum absorbé, ainsi que de cellules de peau morte, de couleur claire, arrachées à la peau par action mécanique de la couche adhésive. L'évaluation quantitative des impuretés visibles sur la surface colorée permet d'évaluer la fréquence nécessaire d'application du patch.

### EXEMPLE II

On réalise une composition comprenant en poids :
69,5% de polymère acrylique en solution dans de l'acétate d'éthyle ;
0,5 % de pigments de bleu de Prusse ;
20 % d'urée ; et
10 % d'acide salicylique.

Le mode opératoire correspond sensiblement à celui décrit dans l'exemple précédent. La matrice adhésive du patch est de couleur bleu foncé.

Un tel patch est particulièrement adapté au traitement de l'acné, l'urée favorisant le dégagement de l'ouverture des glandes sébacées. Il a en outre une action kératolytique, en raison de la présence de l'acide salicylique, favorisant le décollement des cellules de peau morte en surface. L'observation de la face bleue foncée du patch montre la présence de cellules de peau morte ainsi que d'autres résidus, généralement de couleur claire, libérés sous l'action combinée de l'acide salicylique et de l'urée, et prélevées sur la peau par action mécanique de la couche adhésive. De leur nombre dépend la fréquence nécessaire d'application du patch.

### EXEMPLE III

On réalise une solution comprenant en poids :
69,5% de polymère acrylique en solution dans de l'acétate d'éthyle ;
0,5 % de pigments d'oxyde de fer marron ;
20 % d'amidon ; et
10 % d'ORGASOL®.

Le mode opératoire correspond sensiblement à celui décrit dans l'exemple I. La matrice adhésive du patch est de couleur marron.

La combinaison de ces actifs permet d'absorber les constituants gras (sébum) et les constituants hydrophiles sécrétés par la peau, conférant à cette dernière un aspect mat, naturel, et procurant également une grande sensation de douceur. A nouveau, les constituants absorbés par le patch sont visibles, notamment sous formes d'auréoles, sur la face colorée du patch.

## Revendications

1. Patch apte à débarrasser la peau des impuretés qu'elle comporte, comprenant sur un support, une matrice polymérique contenant au moins un actif, la surface du patch destinée à être mise en contact avec la peau étant adhésive, **caractérisée en ce qu'**il comporte une couche colorée de manière à pouvoir, de visu, quantifier et/ou qualifier les impuretés prélevées par ladite surface adhésive.

2. Patch selon la revendication 1 **caractérisé en ce que** l'actif est choisi parmi les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique ; de la poudre de Kaolin, des particules de polyamide (ORGASOL®), des cires, du miel, de la terre de Sienne, des tanins ou des sels minéraux.

3. Patch selon la revendication 1 ou 2 **caractérisé en ce que** la couche colorée est de couleur foncée, de manière à réaliser un contraste suffisant pour mettre en évidence les impuretés extraites de la peau.

4. Patch selon l'une quelconque des revendications 1 à 3 **caractérisé en ce que** la matrice polymérique est formée à base d'un polymère acrylique, vinylique, d'un silicone ou d'un polyuréthanne.

5. Patch selon l'une quelconque des revendications 1 à 4 **caractérisé en ce que** la matrice contient au moins un agent hydroabsorbant, choisi parmi les polyacrylates réticulés superabsorbants, l'alcool polyvinylique, les polymères carboxyvinyliques, les dérivés semi-synthétiques de la cellulose, les amidons, les gommes de guar, arabique ou adragante, la caséine, les phytocolloïdes, les fibres de coton et la gélatine.

6. Patch selon l'une quelconque des revendications 1 à 5 **caractérisé en ce que** le support est un support occlusif.

7. Patch selon la revendication 6 **caractérisé en ce que** le support est constitué d'un matériau thermoplastique, choisi parmi les polyéthylènes haute et basse densité, les polypropylènes, les polychlorure de vinyle, les copolymères d'éthylène et d'acétate de vinyle, les polyesters et les polyuréthannes, ou d'un complexe de tels matériaux.

8. Patch selon l'une quelconque des revendications 1 à 5 **caractérisé en ce que** le support est non occlusif.

9. Patch selon la revendication 8 **caractérisé en ce que** le support est constitué d'un papier, d'un matériau thermoplastique poreux ou perforé, d'un tissé, d'un non tissé, d'un non tissé perforé.

10. Patch selon l'une quelconque des revendications 1 à 9 **caractérisé en ce que** la couche colorée est formée de ladite matrice polymérique.

11. Patch selon la revendication 10 **caractérisé en ce que** la matrice polymérique est colorée par incorporation de pigments colorés.

12. Patch selon la revendication 11 **caractérisé en ce que** les pigments sont choisis parmi les pigments synthétiques, minéraux, notamment des pigments d'oxyde de titane, de zirconium ou de cérium, ainsi que les oxydes de zinc, de fer ou de chrome, et le bleu ferrique ; ou les pigments organiques, notamment le noir de carbone, les laques de baryum, strontium, calcium (DC Red N°7), aluminium, ou un mélange de tels pigments.

13. Patch selon l'une quelconque des revendications 1 à 12 **caractérisé en ce que** la matrice comporte au moins un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

14. Patch selon l'une quelconque des revendications 1 à 13 **caractérisé en ce que** la matrice comporte au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

15. Patch selon l'une quelconque des revendications 1 à 14 **caractérisé en ce qu'**il comprend une feuille de protection, pelable avant application du patch, et imperméable au(x) composé(s) entrant dans la composition de la couche polymérique.

16. Patch selon la revendication 15 **caractérisé en ce que** la feuille de protection est constituée de deux parties se superposant sur une portion médiane du patch.

17. Patch selon l'une quelconque des revendications 1 à 16 **caractérisé en ce qu'**il comprend au moins une zone de faible dimension, apte à former une zone de préhension pour le décollement du patch.

18. Patch selon l'une quelconque des revendications précédentes **caractérisé en ce que** sa durée d'application est comprise entre 30 s et 15 mn, et de préférence, entre 30 s et 5 mn.

19. Patch selon l'une quelconque des revendications précédentes, **caractérisé en ce que** son pouvoir adhésif est compris entre 50 et 800 g/cm², de préférence entre 100 et 700 g/cm², et de préférence encore, entre 300 et 600 g/cm².

20. Patch selon l'une quelconque des revendications précédentes **caractérisé en ce que** sa forme est adaptée à la forme du nez, des lèvres, de la zone comprise entre le nez et la lèvre supérieure, ou du front.

## Patentansprüche

1. Pflaster, das befähigt ist, die Haut von ihren Verunreinigungen zu befreien, das einen Träger und eine Polymermatrix mit mindestens einem Wirkstoff umfaßt, wobei die Oberfläche des Pflasters, die mit der Haut in Kontakt kommen soll, adhäsiv ist, **dadurch gekennzeichnet, daß** es eine farbige Schicht aufweist, um die durch die adhäsive Oberfläche abgelösten Verunreinigungen durch persönlichen Augenschein quantitativ erfassen und/oder näher bestimmen zu können.

2. Pflaster nach Anspruch 1, **dadurch gekennzeichnet, daß** der Wirkstoff unter den Keratolytika, wie beispielsweise den α- und β-Hydroxycarbonsäuren oder β-Ketocarbonsäuren, ihren Salzen, Amiden oder Estern und insbesondere den α-Hydroxysäuren, wie Glykolsäure, Milchsäure, Weinsäure, Äpfelsäure, Citronensäure, Mandelsäure und ganz allgemein den Fruchtsäuren, und den β-Hydroxysäuren, wie Salicylsäure und ihren Derivaten und insbesondere den alkylierten Derivaten, wie 5-n-Octanoylsalicylsäure; Kaolinpulver, Polyamidpartikeln (ORGASOL®), Wachsen, Honig, Terra de Siena, Tanninen oder anorganischen Salzen ausgewählt ist.

3. Pflaster nach Anspruch 1 oder 2, **dadurch gekennzeichnet, daß** die farbige Schicht eine dunkle Farbe aufweist, um einen ausreichenden Kontrast herzustellen und die von der Haut entfernten Verunreinigungen sichtbar zu machen.

4. Pflaster nach einem der Ansprüche 1 bis 3, **dadurch gekennzeichnet, daß** die Polymermatrix auf einem Acrylpolymer, Vinylpolymer, Silicon oder Polyurethan basiert.

5. Pflaster nach einem der Ansprüche 1 bis 4, **dadurch gekennzeichnet, daß** die Matrix mindestens einen Wasserabsorber enthält, der unter den vernetzten Polyacrylat-Superabsorbern, Polyvinylalkohol, Carboxyvinylpolymeren, halbsynthetischen Cellulosederivaten, Stärkeverbindungen, Guar-Gummi, Gummi arabicum, Tragant, Casein, Phytokolloiden, Baumwollfasern und Gelatine ausgewählt ist.

6. Pflaster nach einem der Ansprüche 1 bis 5, **dadurch gekennzeichnet, daß** der Träger ein okklusiver Träger ist.

7. Pflaster nach Anspruch 6, **dadurch gekennzeichnet, daß** der Träger aus einem thermoplastischen Material besteht, das unter den Polyethylenen mit hoher und niedriger Dichte, Polypropylenen, Polyvinylchloriden, Copolymeren von Ethylen und Vinylacetat, Polyestern und Polyurethanen oder Komplexen dieser Materialien ausgewählt ist.

8. Pflaster nach einem der Ansprüche 1 bis 5, **dadurch gekennzeichnet, daß** der Träger nicht okklusiv ist.

9. Pflaster nach Anspruch 8, **dadurch gekennzeichnet, daß** der Träger aus Papier, einem porösen oder perforierten thermoplastischen Material, Gewebe, Vliesstoff oder perforiertem Vliesstoff besteht.

10. Pflaster nach einem der Ansprüche 1 bis 9, **dadurch gekennzeichnet, daß** die farbige Schicht durch die Polymermatrix gebildet wird.

11. Pflaster nach Anspruch 10, **dadurch gekennzeichnet, daß** die Polymermatrix durch Einarbeiten von farbigen Pigmenten farbig ist.

12. Pflaster nach Anspruch 11, **dadurch gekennzeichnet, daß** die Pigmente unter den synthetischen, anorganischen Pigmenten und insbesondere den Pigmenten von Titanoxid, Zirconiumoxid oder Ceroxid, sowie den Oxiden von Zink, Eisen oder Chrom und Eisenblau; oder den anorganischen Pigmenten, insbesondere Ruß, den Lacken von Barium, Strontium, Calcium (DC Red Nr. 7) und Aluminium oder Gemischen dieser Pigmente ausgewählt sind.

13. Pflaster nach einem der Ansprüche 1 bis 12, **dadurch gekennzeichnet, daß** die Matrix mindestens einen wasserlöslichen Wirkstoff enthält, der unter den folgenden Verbindungen ausgewählt ist: Ascorbinsäure und ihren biologisch kompatiblen Salzen, Enzymen, Antibiotika, Verbindungen mit Straffungseffekt, α-Hydroxysäuren und ihren Salzen, hydroxylierten Polysäuren, Saccharose und ihren Derivaten, Harnstoff, Aminosäuren, Oligopeptiden, wasserlöslichen Pflanzenextrakten, Hefeextrakten, Proteinhydrolysaten, Hyaluronsäure, Mucopolysacchariden, den Vitaminen B₂, B₆, H, PP, Panthenol, Folsäure, Acetylsalicylsäure, Allantoin, Glycyrrhetinsäure, Kojisäure und Hydrochinon.

14. Pflaster nach einem der Ansprüche 1 bis 13, **dadurch gekennzeichnet, daß** die Matrix mindestens eine fettlösliche Verbindung enthält, die unter den folgenden Verbindungen ausgewählt ist: D-α-Tocopherol, DL-α-Tocopherol, D-α-Tocopherolacetat, DL-α-Tocopherolacetat, Ascorbylpalmitat, Vitamin F und Glyceriden von Vitamin F, D-Vitaminen, Vitamin D₂, Vitamin D₃, Retinol, Retinolestern, Retinolpalmitat, Retinolpropionat, β-Carotin, D-Panthenol, Farnesol, Farnesylacetat; Jojobaöl und Johannisbeeröl mit einem hohen Anteil an essentiellen Fettsäuren; Keratolytika, wie Salicylsäure, ihren Salzen und ihren Estern, 5-n-Octanoylsalicylsäure und ihren Estern, Alkylestern von α-Hydroxysäuren, wie Citronensäure, Milchsäure und Glykolsäure; Asiatischer Säure, Madekassischer Säure, Asiaticosid, dem Gesamtextrakt von Centella asiatica, β-Glycyrrhetinsäure, α-Bisabolol, Ceramiden, wie 2-Oleoylamino-1,3-octadecan; Phytantriol, Sphingomyelin aus Milch, Phospholipiden mariner Herkunft mit einem hohen Anteil an mehrfach ungesättigten essentiellen Fettsäuren, Ethoxyquin; Rosmarinextrakt, Melissenextrakt, Quercetin, Extrakten aus getrockneten Mikroalgen und steroidalen entzündungshemmenden Wirkstoffen.

15. Pflaster nach einem der Ansprüche 1 bis 14, **dadurch gekennzeichnet, daß** es eine Schutzfolie aufweist, die vor der Anwendung des Pflasters abziehbar und gegenüber der oder den Verbindungen in der Zusammensetzung der Polymerschicht undurchlässig ist.

16. Pflaster nach Anspruch 15, **dadurch gekennzeichnet, daß** die Schutzfolie aus zwei Teilen besteht, die sich im Mittelbereich des Pflasters überlappen.

17. Pflaster nach einem der Ansprüche 1 bis 16, **dadurch gekennzeichnet, daß** es mindestens einen Bereich von geringer Größe aufweist, der für das Ablösen des Pflasters gegriffen werden kann.

18. Pflaster nach einem der vorhergehenden Ansprüche, **dadurch gekennzeichnet, daß** es während einer Zeitdauer von 30 s bis 15 min und vorzugsweise 30 s bis 5 min aufgebracht wird.

19. Pflaster nach einem der vorhergehenden Ansprüche, **dadurch gekennzeichnet, daß** sein Haftvermögen im Bereich von 50 bis 800 g/cm², vorzugsweise 100 bis 700 g/cm² und noch bevorzugter 300 bis 600 g/cm² liegt.

20. Pflaster nach einem der vorhergehenden Ansprüche, **dadurch gekennzeichnet, daß** seine Form an die Form der Nase, der Lippen, den Bereich zwischen Nase und Oberlippe oder der Stirn angepaßt ist.

## Claims

1. Patch, capable of ridding the skin of the impurities which it comprises, comprising, on a backing, a polymeric matrix comprising at least one active principle, the surface of the patch intended to be brought into contact with the skin being adhesive, **characterized in that** it comprises a coloured layer, so as to be able with one's own eyes to quantify and/or qualify the impurities withdrawn by the said adhesive surface.

2. Patch according to Claim 1, **characterized in that** the active principle is chosen from keratolytic agents, such as α- and β-hydroxy-carboxylic or β-ketocarboxylic acids, their salts, amides or esters and more particularly α-hydroxy acids, such as glycolic acid, lactic acid, tartaric acid, malic acid, citric acid or mandelic acid, and generally fruit acids and β-hydroxy acids, such as salicylic acid and its derivatives, in particular alkylated derivatives, such as 5-(n-octanoyl)salicylic acid, kaolin powder, polyamide particles (Orgasol®), waxes, honey, sienna, tannins or inorganic salts.

3. Patch according to Claim 1 or 2, **characterized in that** the coloured layer is dark in colour, so as to achieve a sufficient contrast to demonstrate the impurities extracted from the skin.

4. Patch according to any one of Claims 1 to 3, **characterized in that** the polymeric matrix is formed based on an acrylic or vinyl polymer, on a silicone or on a polyurethane.

5. Patch according to any one of Claims 1 to 4, **characterized in that** the matrix comprises at least one water-absorbing agent chosen from superabsorbent crosslinked polyacrylates, poly(vinyl alcohol), carboxyvinyl polymers, semi-synthetic derivatives of cellulose, starches, guar gum, gum arabic, gum tragacanth, casein, phytocolloids, cotton fibres and gelatin.

6. Patch according to any one of Claims 1 to 5, **characterized in that** the backing is an occlusive backing.

7. Patch according to Claim 6, **characterized in that** the backing is composed of a thermoplastic material, chosen from high- and low-density polyethylenes, polypropylenes, poly(vinyl chloride)s, copolymers of ethylene and of vinyl acetate, polyesters and polyurethanes, or of a complex of such materials.

8. Patch according to any one of Claims 1 to 5, **characterized in that** the backing is non-occlusive.

9. Patch according to Claim 8, **characterized in that** the backing is composed of a paper, of a porous or perforated thermoplastic material, of a fabric, of a nonwoven or of a perforated nonwoven.

10. Patch according to any one of Claims 1 to 9, **characterized in that** the coloured layer is formed of the said polymeric matrix.

11. Patch according to Claim 10, **characterized in that** the polymeric matrix is coloured by incorporation of coloured pigments.

12. Patch according to Claim 11, **characterized in that** the pigments are chosen from synthetic or inorganic pigments, in particular titanium, zirconium or cerium oxide pigments, as well as zinc, iron or chromium oxides and ferric blue, or organic pigments, in particular carbon black or barium, strontium, calcium (DC Red No. 7) or aluminium lakes, or a mixture of such pigments.

13. Patch according to any one of Claims 1 to 12, **characterized in that** the matrix comprises at least one water-soluble active principle chosen from the following compounds: ascorbic acid and its biologically compatible salts, enzymes, antibiotics, components with a tensioning effect, α-hydroxy acids and their salts, hydroxylated polyacids, sucroses and their derivatives, urea, amino acids, oligopeptides, water-soluble plant extracts, yeast extracts, protein hydrolysates, hyaluronic acid, mucopolysaccharides, vitamins B₂, B₆, H and PP, panthenol, folic acid, acetylsalicylic acid, allantoin, glycyrrhetic acid, kojic acid and hydroquinone.

14. Patch according to any one of Claims 1 to 13, **characterized in that** the matrix comprises at least one fat-soluble compound chosen from the following compounds: D-α-tocopherol, DL-α-tocopherol, D-α-tocopheryl acetate, DL-α-tocopheryl acetate, ascorbyl palmitate, vitamin F and vitamin F glycerides, vitamin D, vitamin D₂, vitamin D₃, retinol, retinyl esters, retinyl palmitate, retinyl propionate, β-carotene, D-panthenol, farnesol, farnesyl acetate, jojoba and blackcurrant oils which are rich in essential fatty acids, keratolytic agents, such as salicylic acid, its salts and its esters, 5-(n-octanoyl)salicylic acid and its esters, alkyl esters of α-hydroxy acids, such as citric acid, lactic acid or glycolic acid, asiatic acid, madecassic acid, asiaticoside, total extract of Centella asiatica, β-glycyrrhetinic acid, α-bisabolol, ceramides, such as 2-oleoylamino-1,3-octadecane, phytanetriol, milk sphingomyelin, phospholipids of marine origin which are rich in polyunsaturated essential fatty acids, ethoxyquin, rosemary extract, balm extract, quercetin, extract of dried microalgae, or steroidal anti-inflammatory agents.

15. Patch according to any one of Claims 1 to 14, **characterized in that** it comprises a protective sheet which can be peeled off before application of the patch and which is impermeable to the compound(s) forming part of the composition of the polymeric layer.

16. Patch according to Claim 15, **characterized in that** the protective sheet is composed of two parts which are superimposed over a middle portion of the patch.

17. Patch according to any one of Claims 1 to 16, **characterized in that** it comprises at least one small region capable of forming a gripping region for removal of the patch.

18. Patch according to any one of the preceding claims, **characterized in that** its application time is between 30 s and 15 min and preferably between 30 s and 5 min.

19. Patch according to any one of the preceding claims, **characterized in that** its adhesive power is between 50 and 800 g/cm², preferably between 100 and 700 g/cm² and more preferably between 300 and 600 g/cm².

20. Patch according to any one of the preceding claims, **characterized in that** its shape is suited to the shape of the nose, lips, region between the nose and the upper lip, or forehead.
